(19) Europäisches Patentamt
European Patent Office
Office européen des brevets

(11) **EP 1 803 396 B1**

(12) **EUROPEAN PATENT SPECIFICATION**

(45) Date of publication and mention
of the grant of the patent:
**09.07.2008 Bulletin 2008/28**

(51) Int Cl.:
*A61B 5/11* *(2006.01)*  *A61B 5/024* *(2006.01)*
*G01S 13/02* *(2006.01)*

(21) Application number: **06000082.5**

(22) Date of filing: **03.01.2006**

(54) **Monitoring apparatus for physical movements of a body organ and method for the same**

Apparat und Methode zur Messung von Bewegung von Körperorganen

Appareil et méthode de mesure de mouvement des organes du corps

(84) Designated Contracting States:
**DE FR GB IT**

(43) Date of publication of application:
**04.07.2007 Bulletin 2007/27**

(73) Proprietor: **Industrial Technology Research Institute**
**Judung Township**
**Hsin chu 310 (TW)**

(72) Inventors:
• **Tao, Teh Ho**
**Hsinchu City (TW)**

• **Peng, Jia Hung**
**Hsinchu City 300 (TW)**
• **Wong, Yao Lung**
**Sinjhuang City**
**Taipei, County 242 (TW)**

(74) Representative: **Liesegang, Roland**
**FORRESTER & BOEHMERT**
**Pettenkoferstrasse 20-22**
**80336 München (DE)**

(56) References cited:
WO-A-20/05092190  GB-A- 2 349 759
US-A- 4 926 868  US-A- 5 394 882
US-A- 5 573 012

**Description**

**BACKGROUND OF THE INVENTION**

(A) Field of the Invention

The present invention relates to a monitoring apparatus for physical movements of a body organ and method for the same, and more particularly to a monitoring apparatus for physical movements of a body organ using ultra wideband (UWB) electromagnetic wave technology to detect the physical movements of a body organ and method for the same.

(B) Description of the Related Art

The purpose of monitoring patient's vital signs, such as heart beat and arterial pulse, is to alert the patient or his/her caregivers of any abnormal condition of the cardiovascular system. Conventional technologies for monitoring patients' arterial pulses most commonly employ piezoelectric pressure sensors to obtain signals at points on the body where arterial blood vessels 26 are located. US 4,489,731 discloses a wrist-worn device using a piezoelectric crystal as the detector for sonic vibrations caused by arterial pulses. US 5,795,300 discloses a device containing piezoelectric sensing elements preferably positioned over the radial artery in the subpollex depression parallel to the tendon cord bundles on the volar surface of the wrist so as to maximize the signal-to-noise ratio. Most commonly used technology for monitoring heartbeat utilizes skin electrodes to acquire electrocardiogram signals. During operation, either pressure sensor or skin electrode must be pressed against the patient's skin so that a signal with good quality can be obtained. However, it may cause patient discomfort in long-term operation due to prolonged compression of skin tissue. Furthermore, skin irritation and sweating may be induced, which results in signal-to-noise ratio degradation and signal baseline drift.

US 5,573,012 uses the ultra wideband (UWB) electromagnetic means to detect movements of the heart, lungs and vocal cords. The device is designed to use the non-acoustic pulse radar monitoring employed in the repetitive mode, whereby a large number of reflected pulses are averaged. It incorporates a time-gate scheme of which time gating pulses are generated by a range delay generator. The time gating pulses cause the receiving path to selectively conduct pulses reflected from the body parts and received by a receiver antenna. The device converts the detected voltage into an audible signal using both amplitude modulation and the Doppler effect. The device is designed for remote operation and thus cannot be attached to the patient under examination. Therefore, it is not suitable for portable long-term monitoring purposes.

In view of the aforementioned problems, there is a need for a monitoring apparatus, which can be worn by patients and provides the characteristics of long-term stable operation, small size and low cost.

**SUMMARY OF THE INVENTION**

The objective of the present invention is to provide a monitoring apparatus for physical movements of a body organ using ultra wideband electromagnetic wave to detect physical movements of the body organ and method for the same.

In order to achieve the above-mentioned objective, and avoid the problems of the prior art, the present invention provides a monitoring apparatus for physical movements of a body organ comprising an antenna board including a transmission antenna for radiating ultra wideband electromagnetic waves (probing pulses) to a body organ and a reception antenna for receiving the ultra wideband electromagnetic waves scattered by the body organ, an analog board having a plurality of electronic devices for acquiring analog signals representing the physical movement of the body organ, a digital board having a plurality of electronic devices for digitalizing the analog signal, and a display device for showing the physical movement of the body organ. Particularly, the electronic devices on the analog board comprise a first pulse generator configured to produce the ultra wideband electromagnetic probing pulses, a second pulse generator configured to produce reference pulses, and a balanced mixer having a first input port electrically connected to the second pulse generator and a second input port electrically connected to the reception antenna for producing a phase difference signal representing the physical movements of the body organ.

The present method for acquiring the physical movement of the body organ first radiates a series of ultra wideband electromagnetic pulses (probing pulses) to the body organ. Secondly, the variation of phase differences $\Delta\varphi$ between the probing pulses scattered by the physical movement of the body organ and reference pulses are measured according to the following equations:

$$\Delta\varphi = \Delta\varphi_1 - \Delta\varphi_2 = \frac{4\pi \cdot \sqrt{\varepsilon} \cdot f}{c}\Delta d$$

$$\Delta d = V\,T_a$$

Wherein *f* represents the repetition frequency of the probing pulses, *c* represents velocity of light and $\varepsilon$ represents the relative dielectric constant of the human skin tissue, *V* represents the velocity of the physical movement of the body organ toward where the probing pulses are radiated from, $T_a$ represents the time interval as the body organ moves a distance $\Delta d$. Therefore, the physical movement of the body organ $\Delta d$ is linearly proportional to the phase difference $\Delta\varphi$.

**BRIEF DESCRIPTION OF THE DRAWINGS**

Other objectives and advantages of the present invention will become apparent upon reading the following descriptions and upon reference to the accompanying drawings in which:

FIGs. 1(a), 1(b) and 1(c) show the monitoring apparatus of the present invention attached onto a human body at points where movements of arterial blood vessels can be detected;

FIGs. 2(a) and 2(b) show the monitoring apparatus according to the present invention;

FIG. 3 (a) shows the time diagram of a probing pulse;

FIG. 3(b) shows the frequency spectrum of a radiated probing pulse;

FIG. 4 is a functional block diagram of the monitoring apparatus according to the present invention;

FIGs. 5(a), 5(b), 5(c) and 5(d) are circuit diagrams of the analog board according to one embodiment of the present invention;

FIG. 6(a) and 6(b) illustrate the operation of the balanced mixer according to the present invention;

FIG. 7(a) shows the timing relationship of the received probing pulses and the reference pulses at the balanced mixer;

FIG. 7(b) shows time diagrams of signals at the output of the balanced mixer and of the first low-pass filter;

FIG. 8(a) shows the signal time diagram at the output of the first low-pass filter when the movement of the arterial blood vessel is not detected;

FIG. 8(b) shows the time diagram of the signal at the output of the first low-pass filter when the movement of the arterial blood vessel is detected;

FIG. 9 shows the frequency spectrum of the balanced mixer's output signal according to the present invention;

FIG. 10 shows both the signal spectrum envelope inputting to the transmission antenna and antenna's frequency performance according to the present invention;

FIG. 11 shows a circuit diagram of the digital board according to the present invention;

FIG. 12(a) shows a loop antenna suitable for the monitoring apparatus according to the present invention;

FIG. 12(b) shows a bow-tie antenna suitable for the monitoring apparatus according to the present invention;

FIG. 12(c) shows a terminating half-wave antenna suitable for the monitoring apparatus according to the present invention;

FIG. 12(d) shows a spiral antenna suitable for the monitoring apparatus according to the present invention;

FIGs. 13(a) and FIG. 13(b) show the comparison of radial arterial signal waveform using the monitoring apparatus of the present invention and the electrocardiogram signal waveform.

FIG. 14 shows a portion of an analog board for the monitoring apparatus according to another embodiment of the present invention;

FIG. 15(a) shows the bandwidth of the monitoring apparatus using a single-ended output reception antenna;

FIG. 15(b) shows the bandwidth of the monitoring apparatus using a differential output reception antenna;

FIG. 16(a) shows the radial arterial signal waveform acquired by the present monitoring apparatus using a single-ended output reception antenna;

FIG. 16(b) shows the radial arterial signal waveform acquired by the present monitoring apparatus using a differential output reception antenna;

FIG. 17(a) illustrates a flexible slot antenna according to another embodiment of the present invention;

FIG. 17(b) illustrates a flexible antenna according to another embodiment of the present invention;

FIG. 18(a) shows the bandwidth of the flexible slot antenna;

FIG. 18(b) shows the bandwidth of the flexible antenna;

FIG. 19(a) shows the radial arterial signal waveform acquired by the present monitoring apparatus 10 using the flexible slot antenna; and

FIG. 19(b) shows the radial arterial signal waveform acquired by the present monitoring apparatus 10 using the flexible antenna.

## DETAILED DESCRIPTION OF THE INVENTION

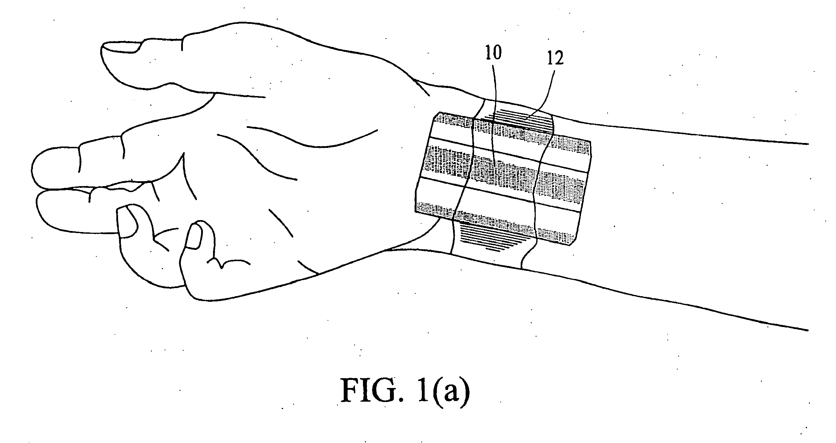

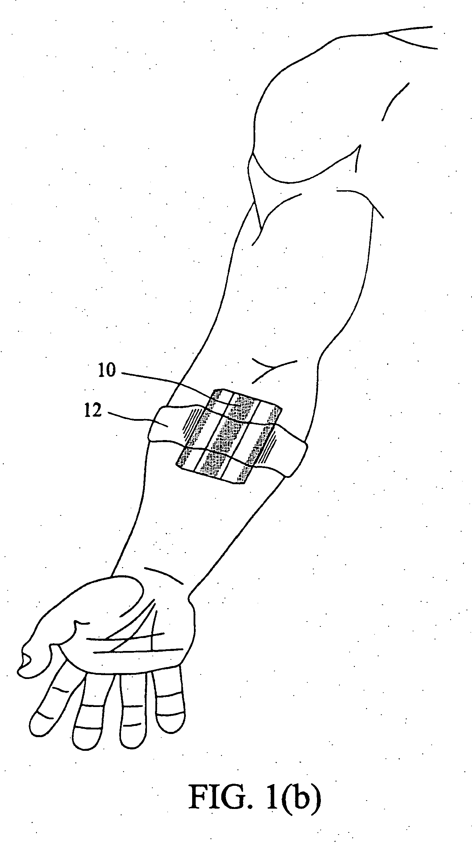

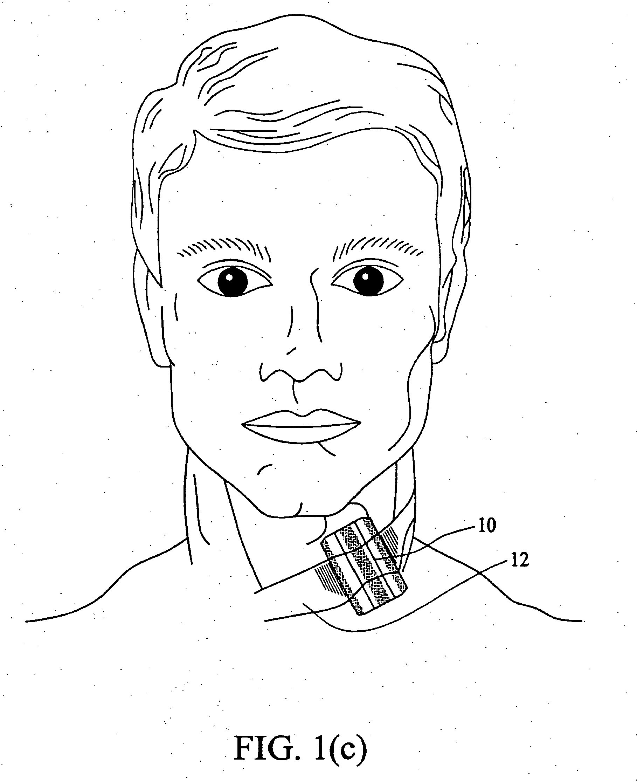

FIGs. 1(a), 1(b) and 1(c) show the monitoring apparatus 10 of the present invention attached onto the human body at points where physical movement of arterial blood vessels can be detected. However, it should be clear that the monitoring apparatus 10 could also be used in a variety of other applications. For example, the monitoring apparatus 10 can be attached onto other points of the body, including but not limited to a heart behind a chest wall, a fetus, the vocal chords, muscle, etc. For simplicity of illustration the object will be exemplified by arterial blood vessels. The strap 12 is used for attaching the monitoring apparatus 10 onto body points without exerting pressure on the skin of the patient.

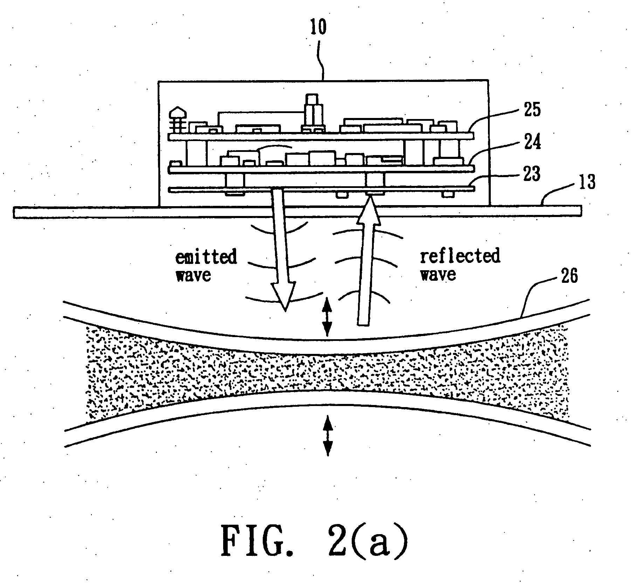

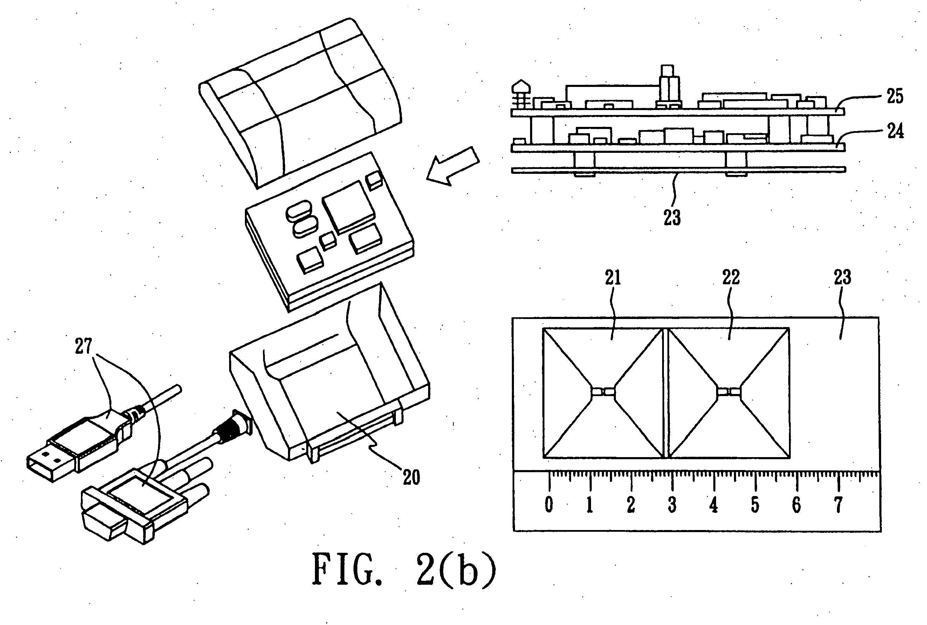

FIGs. 2(a) and 2(b) show the monitoring apparatus 10 according to one embodiment of the present invention. As shown in FIG. 2(a), the monitoring apparatus 10 comprises an antenna board 23, an analog board 24 and a digital board 25. The antenna board 23 can emit waves to an arterial blood vessel 26 and receives waves reflected from it. As shown in FIG. 2(b), the antenna board 23 comprises a transmission antenna 21 and a reception antenna 22 from which a series of probing pulses are emitted and received through a membrane 20. The transmission antenna 21 and the reception antenna 22 are both in the form of bow-tie antenna. The membrane 20 does not serve the purpose of signal energy conversion but acts as a part of the enclosure for the monitoring apparatus 10. Therefore, its material property should not cause attenuation of the energy of the probing pulses. The membrane 20 is preferably made from polymeric materials such as silicone rubber or polycarbonate with a thickness between 0.2 and 0.5 mm.

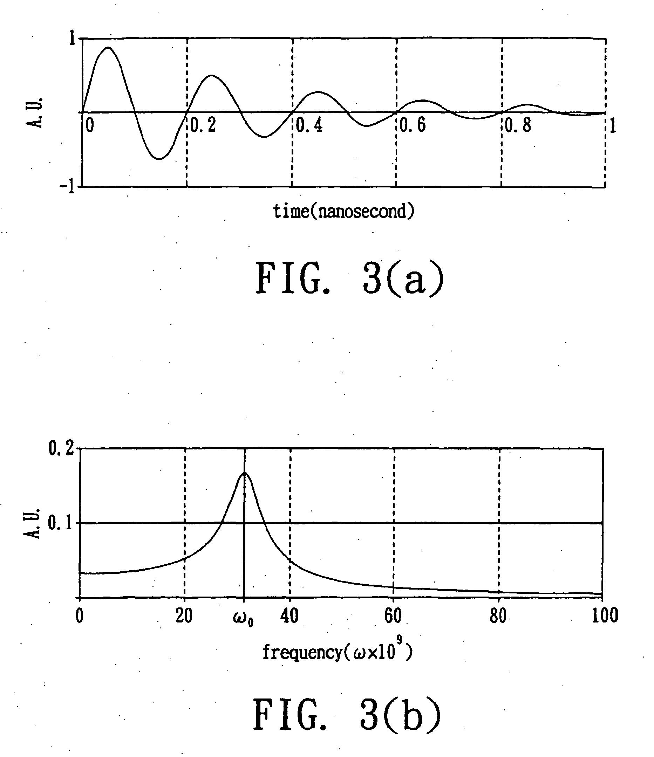

FIG. 3(a) shows the time diagram of the probing pulse. Each probing pulse consists of damped sinusoidal oscillations with a resonance frequency determined by the physical size of the transmission antenna. FIG. 3(b) shows the frequency spectrum of the probing pulse. The center frequency and the bandwidth are determined by the duration of the damped sinusoidal oscillations. When the duration of the damped sinusoidal oscillations approaches zero (ideal shape of an impulse), the center frequency and the bandwidth of the frequency spectrum approach infinity.

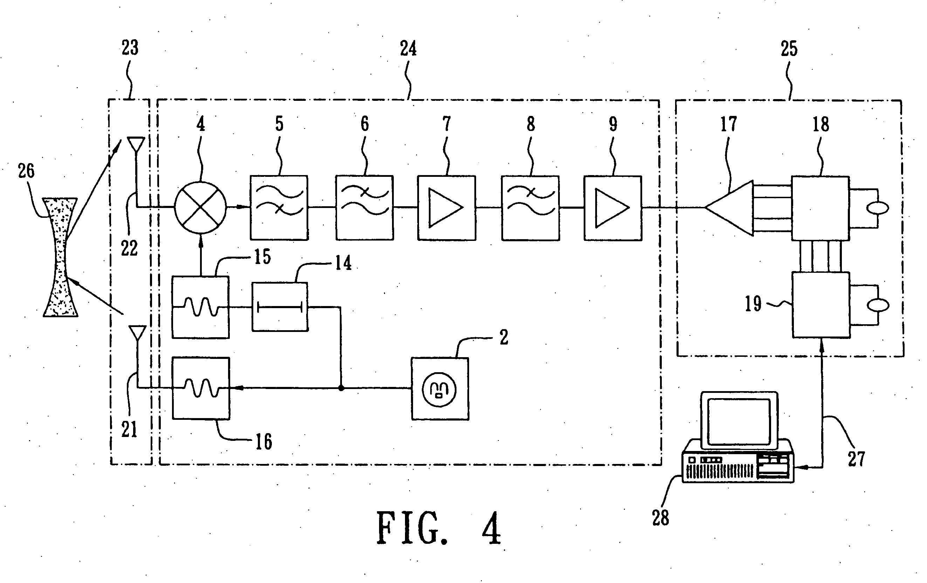

FIG. 4 is a functional block diagram of the monitoring apparatus 10 according to the present invention. The transmission antenna 21 and the reception antenna 22 are positioned on the antenna board 23, and both are performed on the basis of broadband dipole antenna (bow-tie antenna). The analog board 24 comprises a balanced mixer 4 connected to the reception antenna 22, a high-pass filter 5 connected to the balanced mixer 4, a first low-pass filter 6 connected to the high-pass filter 5, a first amplifier 7 connected to the first low-pass filter 6, a second low-pass filter 8 connected to the first amplifier 7, a second amplifier 9 connected to the second low-pass filter 8, a first pulse generator 15 connected to the balanced mixer 4, a delay line 14 connected to the first pulse generator 15, a second pulse generator 16 connected to the transmission antenna 21, and a clock generator 2 connected to the second pulse generator 16 and the delay line 14. The digital board 25 comprises a microcontroller 18 with an embedded analog-to-digital converter 17 and universal asynchronous transceiver 19.

Analog signal processing circuits on the analog board 24 separate a signal representing the physical movements of the arterial blood vessel 26 from other spectral components due to scattering from tissue surrounding the arterial blood vessel 26. The detected signals are then digitized using the microcontroller 18 and transmitted using the transceiver 19 in the digital board 25. The digitized signals can be transmitted through RS232 or USB cable 27 to an external data processing and display unit 28.

The UWB electromagnetic wave is selected due to the following advantages:

1. Reduction in the spectral density of emission power. This makes it possible to lower the level of electromagnetic emission, which influences both doctor and patient, as well as the level of electromagnetic interference with other hospital equipment.

2. Achievement of reduction in the device's overall size.

3. Increase in the device's immunity to external interferences and improvement in the reliability of measurement.

As constructing conventional narrow-band radars, the present monitoring apparatus (a UWB radar) uses the property of electromagnetic wave to be scattered from a boundary of two media with different parameters, which is well-known from the general theory. The electromagnetic pulses radiated by radar are scattered by a moving object. In this case, the oscillation frequency $f$ within the scattered pulses changes owing to the Doppler effect. As frequency variation leads to variations of oscillation period $T$, the number of oscillations in the scattered pulses remains the same. Consequently, the duration ($\tau$) of the scattered pulses is changed. Due to the same effect, the repetition frequency of the electromagnetic pulses scattered by the object $F_r$ and, correspondingly, the repetition period $T_r$ also changes simultaneously. The sign of these variations depends on the direction of target movement relative to the radar, and the variation value depends on the object's radial velocity.

Nevertheless, it should be noted that the usage of these effects in UWB radars, which are intended for detecting and measuring parameters of moving targets, has some specific features. In general cases, to separate such a signal, variations of all three parameters of the pulse sequence mentioned above can be used. However, the oscillation frequency variations within a single scattered pulse are rather small because the duration of a single scattered pulse is very short. For example, for pulse duration $\tau$ = 0.2 nanoseconds, it does not contain one period of oscillation with frequency of 1 GHz. Therefore, it is impossible to determine variation of frequency within a single scattered pulse using conventional filtering techniques.

However, it is possible to make an attempt to measure the phase difference, which appears between the series of scattered and radiated pulses. The phase difference can be estimated as follows. When the object is moving towards the radar with a radial velocity $V$, the repetition period of the scattered pulses changes and becomes

$$T_0 = T\left(1 - \frac{V}{c}\right) \qquad\qquad (1)$$

Where c is the velocity of light. The phase difference and peculiarities of its variations during target movement can be determined as follows. If the instant value of the phase of the series of radiated pulses is $\phi_u(t)=2\pi f t$, where $f$ represents the repetition frequency of the radiated pulses, then the instant phase value for the series of pulses scattered by a stationary object located at a distance $R$ is equal to

$$\varphi_o(t) = \varphi_u(t) + 2\pi \cdot f \frac{2R}{c} = 2\pi \cdot f\left(t + \frac{2R}{c}\right) \qquad\qquad (2)$$

The phase difference between radiated and scattered pulses can be expressed as follows:

$$\Delta\varphi = \varphi_u(t) - \varphi_o(t) = -4\pi \cdot f\, \frac{R}{c} \qquad\qquad (3)$$

From Eq.(3), the phase difference between the series of radiated and scattered pulses due to physical movement of an arterial blood vessel 26 can be derived as follows. If the arterial blood vessel 26 is located at a distance $R_1 = D_0 + D_1$, where $D_0$ represents the distance between the surfaces of the antenna board 23 and that of the skin 13, $D_1$ represents the distance between the skin 13 and the arterial blood vessel 26, then the instant phase of the series of scattered pulses is:

$$\varphi_{o1}(t) = \varphi_u(t) + 2\pi \cdot f\, \frac{2(D_0 + D_1\sqrt{\varepsilon})}{c} = 2\pi \cdot f\left( t + \frac{2(D_0 + D_1\sqrt{\varepsilon})}{c} \right) \quad (4)$$

The phase difference between radiated and scattered pulses will be:

$$\Delta\varphi_1 = \varphi_u(t) - \varphi_{o1}(t) = -4\pi \cdot f\, \frac{(D_0 + D_1\sqrt{\varepsilon})}{c} \qquad\qquad (5)$$

If the arterial vessel moves to a distance $R_2 = D_0 + D_2$ from the radar, then the instance phase of the series of scattered pulses is:

$$\varphi_{o2}(t) = \varphi_u(t) + 2\pi \cdot f\, \frac{2(D_0 + D_2\sqrt{\varepsilon})}{c} = 2\pi \cdot f\left( t + \frac{2(D_0 + D_2\sqrt{\varepsilon})}{c} \right) \quad (6)$$

The phase difference between radiated and scattered pulses will have another value:

$$\Delta\varphi_2 = \varphi_u(t) - \varphi_{o2}(t) = -4\pi \cdot f\, \frac{(D_0 + D_2\sqrt{\varepsilon})}{c} \qquad\qquad (7)$$

Subtracting Eq.(7) from Eq.(5), the variation of the phase difference caused by the movement of arterial blood vessel 26 is obtained, which is:

$$\Delta\varphi(t) = \Delta\varphi_1 - \Delta\varphi_2 = -\frac{4\pi \cdot f \cdot \sqrt{\varepsilon}}{c}(D_1 - D_2) = -\frac{4\pi \cdot f \cdot \sqrt{\varepsilon}}{c} V T_a \quad (8)$$

Consequently, the phase difference $\Delta\varphi$ varies from period to period and this variation depends on the velocity $V$ and the moving period of oscillation $T_a$ of the arterial blood vessel 26. With the repetition frequency of the radiated pulses $f = 10$ MHz, $\varepsilon = 40$ and the physical movement of the arterial blood vessel 26 $D_1 - D_2 = 2.0$ millimeters, the variation of the phase difference $\Delta_\phi = 0.3$ degree, which allows detection of the phase difference using conventional phase measurement devices is obtained.

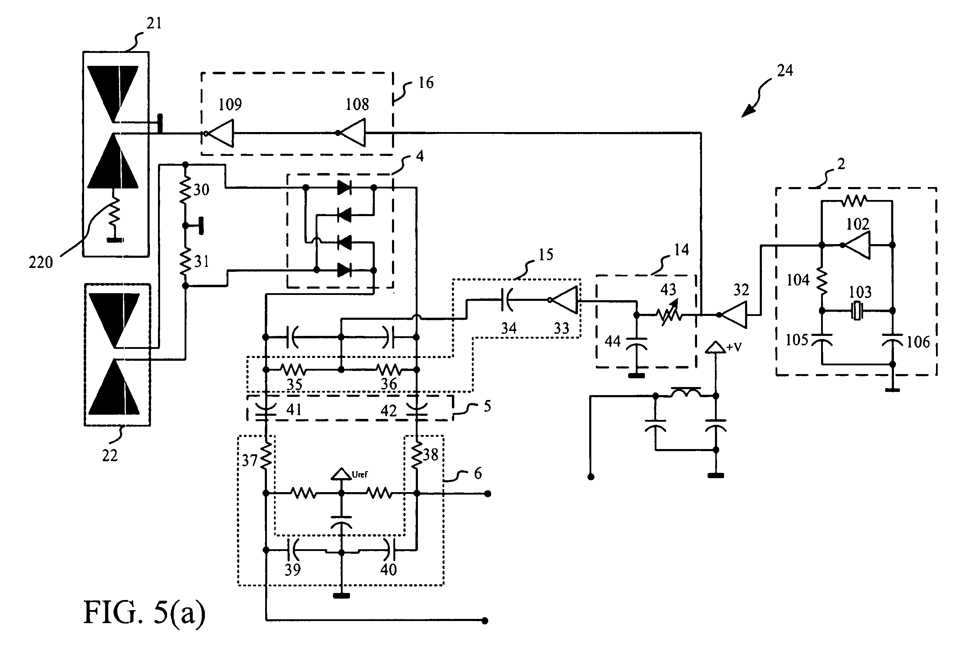

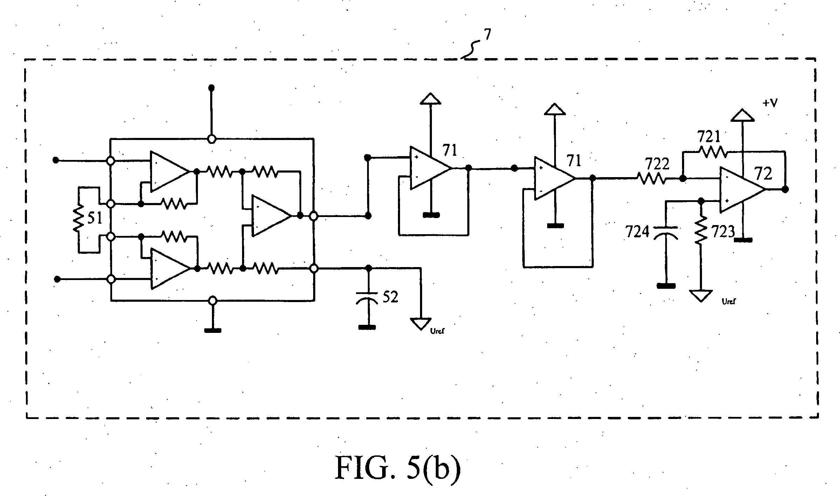

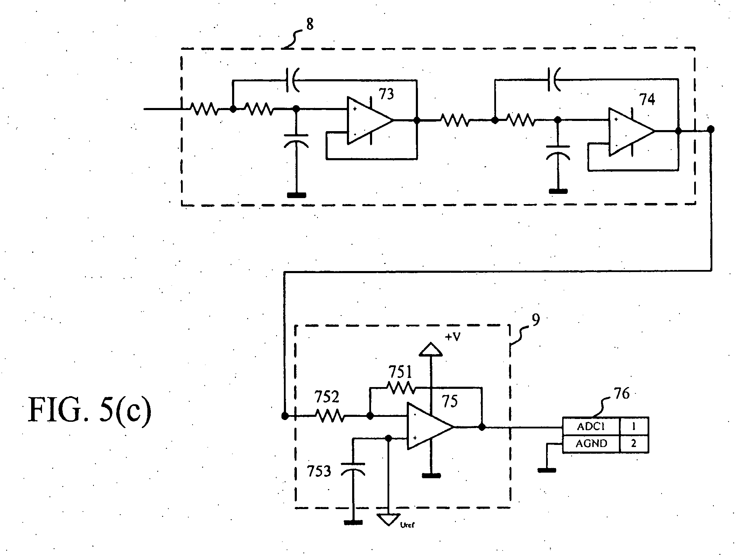

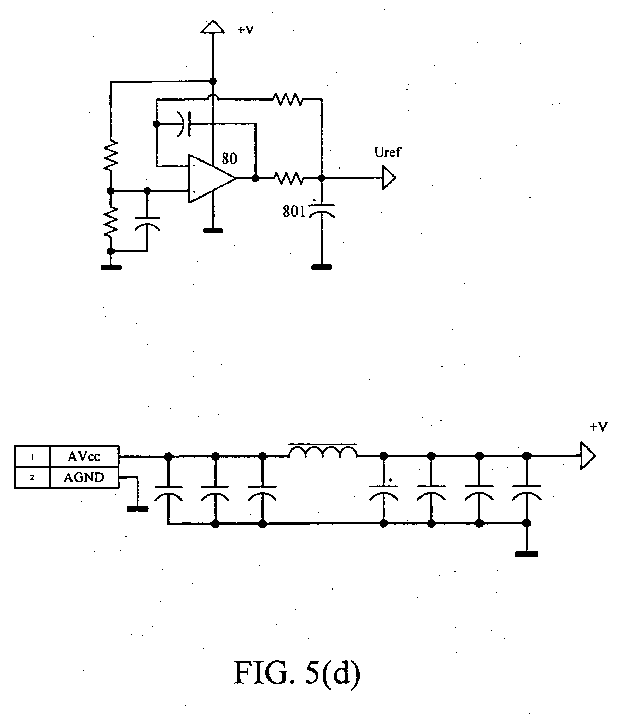

The operation of the monitoring apparatus 10 will be described in detail below. FIGs. 5(a), 5(b), 5(c) and 5(d) are circuit diagrams of the analog board 24 according to one embodiment of the present invention. The clock generator 2, realized on a logic inverter 102, produces square pulses and synchronizes the operation of the analog signal processing circuits on the analog board 24. The timing accuracy of the clock generator 2 is determined by the quartz crystal 103. Low cost crystals are available with an accuracy of $\pm30$ppm. A resistor 104 buffers the quartz crystal 103 from sharp logic transitions and prevents spurious oscillation modes. The combination of capacitors 105 and 106 forms an approximate load capacitance as specified for the quartz crystal 103. The resistor 104 provides a negative resistive feedback to bias the inverter 102 at its threshold (on average) and AC feedback through the quartz crystal 103 to control the oscillating frequency.

The second pulse generator 16 (a shaper of the transmitter's probing pulse) consisting of logic inverters 108 and 109 is connected to the transmission antenna 21. A pull-up resistor 220 (50 Ohm) is connected to the edge of one vibrator of transmission antenna 21 to reduce duration of oscillations ("ring") at the transmission antenna 21. The clock signal enters the receiver circuits via a buffer, realized on a logic inverter 32, which reduces the influence of the receiver to the operation of transmitting circuits and the clock generator 2. Reference pulses are formed from the delayed clock signal from the clock generator 2 and go to the junction of the resistors 35 and 36 after they are shaped into short pulses by the first pulse generator 15 consisting of logic inverter 33, capacitor 34 and resistors 35, 36. The purpose of the delay line 14, consisting of a variable resistor 43 and a capacitor 44, is to match the timing between the radiated probing pulses and the reference pulses so that their phase differences can be correctly measured at the balanced mixer 4. The time delay of the reference pulses ($T_{del}$) is determined according to the following formula:

$$T_{del} = 2\left( \frac{D_0}{c} + \frac{D_1}{c} \cdot \sqrt{\varepsilon} \right) \tag{9}$$

Where $D_0$ represents the distance between the surface of the antenna board 23 and the skin 13, $D_1$ represents the distance between the skin 13 and the arterial blood vessel 26, $c$ represents the velocity of light, and $\varepsilon$ represents the relative dielectric constant of human skin tissue. As shown in FIG. 5(a), $T_{del}$=1.204RC, where RC is the product of the resistance of the variable resistor 43 and the capacitance of the capacitor 44, which constitute the delay line 14. Particularly, $T_{del}$ can be adjusted by changing the resistance of the variable resistor 43. Accepted by the reception antenna 22, the probing pulses proceed to the input contacts of the balanced mixer 4. The resistors 30 and 31 are matched loads for the symmetric reception antenna 22.

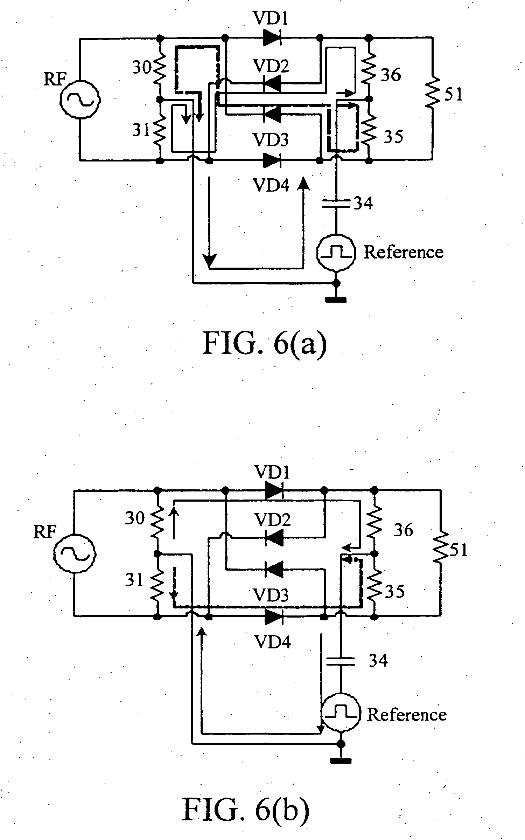

FIG. 6(a) and 6(b) illustrate the operation of the balanced mixer 4. During the positive half-period of the reference pulse, diodes VD2, VD3 are conductive and the received probing pulse proceeds to the output of the balanced mixer 4 as shown in FIG. 6(a). During the negative half-period of reference pulse (FIG. 6(b)), diodes VD1, VD4 are conducting and the probing pulse proceeds to the output of the balanced mixer 4 with a phase shift of 180 degrees. Accordingly the output voltage of the balanced mixer 4 is defined by the following expression:

$$U_{LOAD} = R(I_{VD1} - I_{VD4}) + R(I_{VD2} - I_{VD3}) \tag{10}$$

Where $R$ represents the resistance of the resistors 35 and 36. The voltage-current characteristic of the diode can be approximated by a polynomial of the third degree $I_{VD} = a_o + a_1U + a_2U^2 + a_3U^3$. Substituting this expression into equation (10), two equations can be obtained as follows:

$$R(I_{VD1} - I_{VD4}) = 2R\left( a_1 \frac{U_{RF}}{2} + 2a_2 \frac{U_{RF}}{2} U_{LO} + a_3 \frac{U_{RF}^3}{2} + 3a_3 U_{LO}^2 \frac{U_{RF}}{2} \right) \tag{11}$$

$$R(I_{VD2} - I_{VD3}) = 2R\left( -a_1 \frac{U_{RF}}{2} + 2a_2 \frac{U_{RF}}{2} U_{LO} - a_3 \frac{U_{RF}^3}{2} - 3a_3 U_{LO}^2 \frac{U_{RF}}{2} \right) \tag{12}$$

Adding equations (11) and (12),

$$U_{LOAD} = 4Ra_2 U_{RF} U_{LO} \tag{13}$$

From Eq.(13), it shows that the balanced mixer 4 realizes multiplication of received probing pulses $U_{RF}$ with the reference pulses $U_{LO}$.

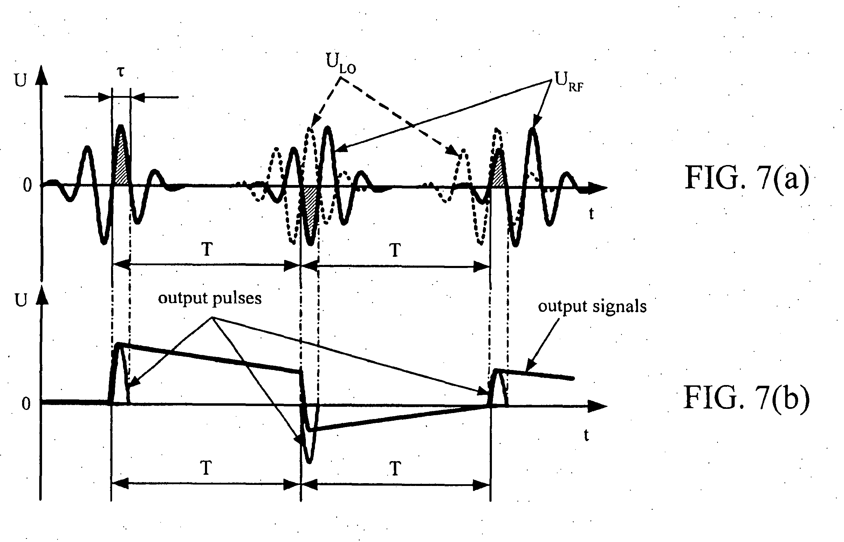

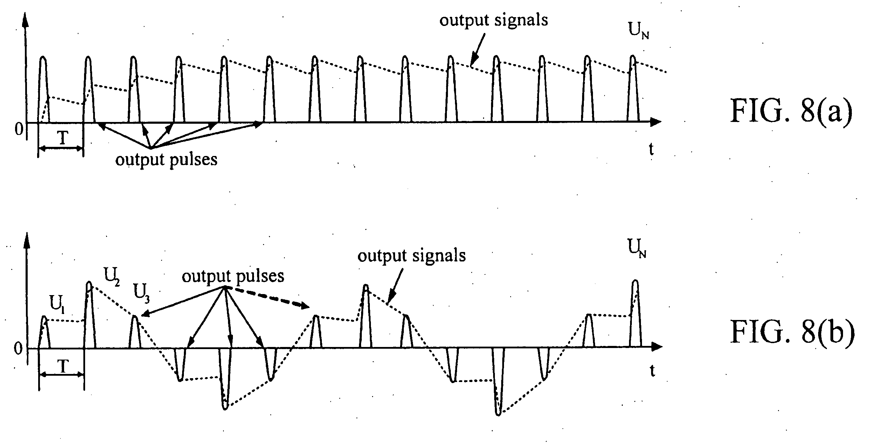

FIG. 7(a) shows the time diagrams of the received probing pulses and the reference pulses at the balanced mixer 4, wherein the received probing pulses $U_{RF}$ are shown by the solid line and the reference pulses $U_{LO}$ are shown by the dashed line. As the arterial blood vessel 26 moves toward and away from the monitoring apparatus 10, the corresponding time variation of phase difference (FIG. 7(a)) results in positive and negative pulses at the output of the balanced mixer 4 (FIG. 7(b)). As a result, the output signals will be generated at the output of the first low pass filter 6. FIG. 8(a) and FIG. 8(b) further illustrate the comparison between the time diagrams for a motionless arterial blood vessel and for moving arterial blood vessel 26, respectively.

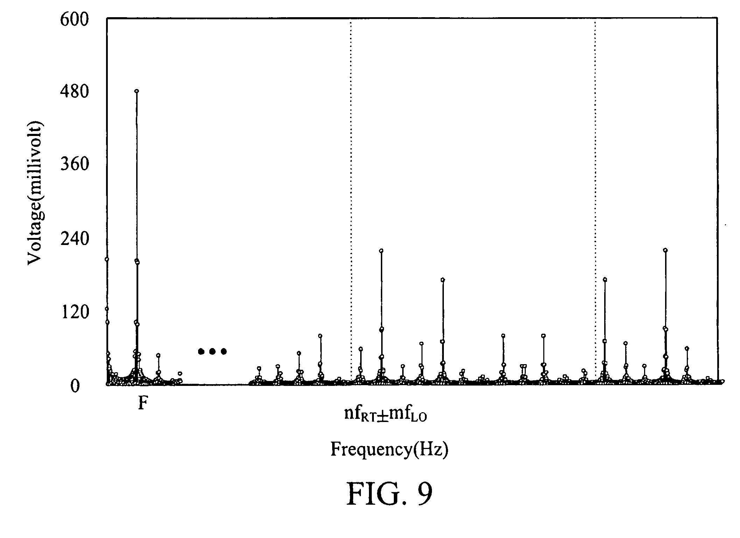

The output pulses at the balanced mixer 4 proceed to the first low-pass filter 6 for filtering of undesired high frequency components to generate output signals. As shown in FIG. 9, the peaks at frequencies. $nf_{RF} \pm mf_{LO}$ represent repetitive frequency $f_{RF}$ of the received probing pulses and the repetitive frequency $f_{LO}$ of the reference pulses respectively. These high frequency components are filtered out by the first low-pass filter 6, while the frequency component F, representing the signal modulated by the physical movement of the arterial blood vessel 26, is selected.

Application of the balanced mixer 4 allows greater precision in making the operation of multiplication between input and reference pulses. In other words, a greatly decreased number of mixer output signal distortions can be obtained. The balanced circuit configuration allows a high isolation between input signal (from the antenna) and reference input, and between these inputs and the mixer's output (mean of isolation is about 40dB), thereby noticeably reducing infiltration of reference pulses to the input of balanced mixer 4 and radiation by the reception antenna 22.

Referring back to FIG. 5(a) again, the high-pass filter 5 consisting of capacitors 41 and 42 removes the DC component, which is formed because of scattered signals from the stationary tissue surrounding the blood vessel 26. The first low-pass filter 6, which consists of resistor 38, capacitor 40, resistor 37 and capacitor 39, is used to select a low frequency signal corresponding to the movement of the arterial blood vessel 26.

As shown in FIG. 5(b), the low frequency signal is then amplified by the first amplifier 7, which consists of a first stage amplification (a low-frequency instrumentation amplifier) and a second stage amplification. Gain of the first amplification is set at 117.28 (41.38 dB) by adjusting the resistance of the resistor 51. The first stage amplification also provides suppression of common-mode interference not less than 110 dB.

The signal enters the second stage of amplification, on the basis of operational amplifiers 71 and 72. Gain of the second stage is adjusted by resistors 721 and 722, and is determined by the following formula:

$$K2 = -\left[\frac{R(721)}{R(722)}\right] = -\left[\frac{100KOhm}{10KOhm}\right] = -10 \quad (20dB)$$

The resistance of the resistor 723 is determined by the following formula:

$$R(723) = \frac{R(721) \cdot R(722)}{R(721) + R(722)} = \frac{1000}{110} = 9.1KOhm$$

Referring to FIG. 5(c), amplified signals enter the second low-pass filter 8 (the fourth-order Butterworth active filter) based on the operational amplifiers 73 and 74. This type of filter provides the most uniform frequency response within the pass band.

The filtered signal goes into the third stage amplification at the second amplifier 9 based on the operational amplifier 75. Gain of the third stage is adjusted by resistors 751 and 752 and is determined by the following formula:

$$K3 = -\left(\frac{R(751)}{R(752)}\right) = -\left(\frac{4.7kOhm}{2kOhm}\right) = -2.35 \quad (7.4 \text{ dB})$$

Total gain of the receiver is equal to:

$$K = K1K2K3 = 117.28 \cdot 10 \cdot 2.35 = 2756.08 \quad (68.8 \text{ dB})$$

Referring to FIG. 5(d), virtual-ground is created using a voltage divider buffered by an operational amplifier 80. This circuit will generate a virtual-ground reference at 1/2 of the supply voltage. The circuit includes compensation to allow for bypass capacitors 801, 52, 724, and 753 at the virtual-ground output. The benefit of a large capacitor is that not only does the virtual-ground present a very low DC resistance to the load, but its AC impedance is low as well. The operation amplifier 80 should both sink and source more than 5 mA, which improves recovery time from transients in the load current.

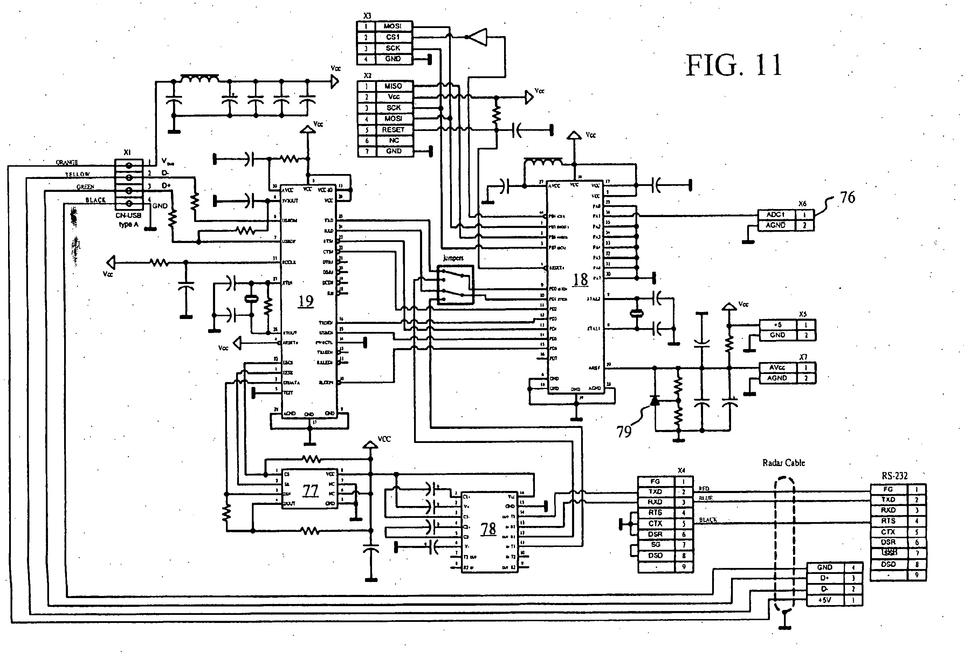

FIG. 11 shows a circuit diagram of the digital board 25 according to the present invention. The amplified, filtered signal is transmitted from the analog board 24 via a connector 76 (FIG. 5(c)) to an analog-to-digital converter embedded in the microcontroller 18. The microcontroller 18 is preferably a low-power CMOS 8-bit microcontroller based on the RISC architecture. It carries out data collection and data transmission from the analog signal processing circuits. Data transmission can be carried out either by RS-232interface or by interface USB. Driver of COM-port 78 is used to connect the digital board 25 with an external data processing and display unit 28 by RS-232 interface. The transceiver 19 and memory EEPROM 77 are used for communication via interface USB. The transceiver 19 is a single chip USB UART (U-UART) for transferring serial data over USB with data transfer rates up to 920k baud. The EEPROM 77 required for storage of the configurable parameters includes the USB Vendor ID (VID), Product ID (PID), Serial Number and Strings of the controller. Source of the reference voltage on the basis of the three-terminal adjustable shunt regulator 79 is installed to supply analog signal processing circuits of the analog board 23 and generation of reference voltage of microcontroller's ADC in the digital board 25.

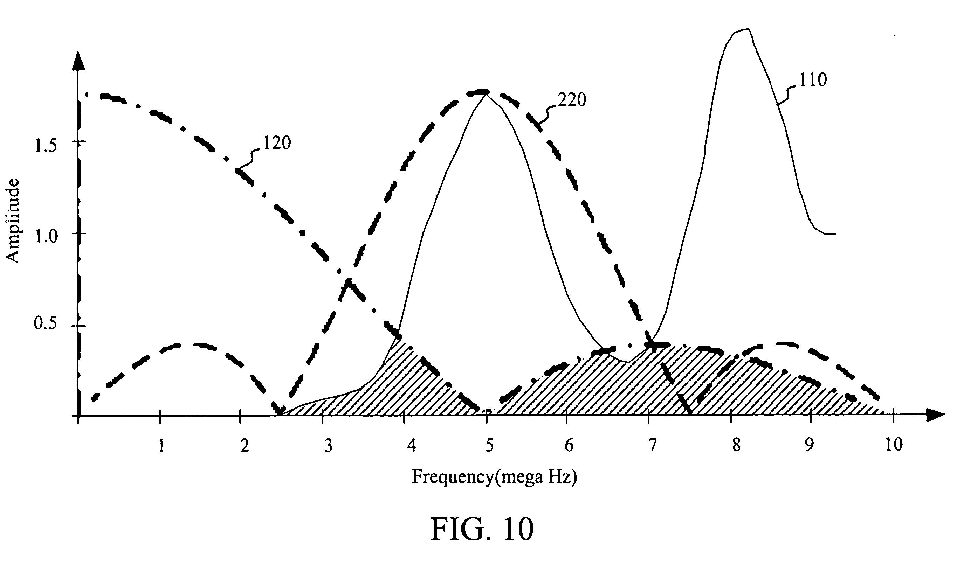

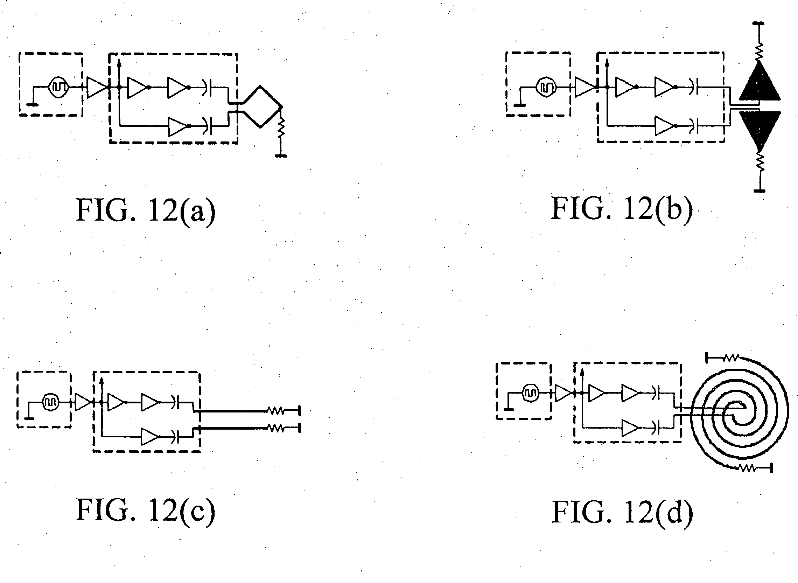

Referring to FIG. 10, improvement in the energy performance of the present monitoring apparatus 10 is due to matching between the signal amplitude spectrum 220 into the transmission antenna 21 and the frequency performance 110 of the transmission antenna 21. As a result, the radiated signal energy is nearly twice as much as that of the conventional device with signal spectrum envelope 120 and identical antenna frequency performance. Matching between signal amplitude spectrum and antenna's frequency performance can be achieved by a suitable selection of transmission antennas such as loop antenna, a bow-tie antenna, a terminating half-wave antenna and a spiral antenna, as shown in FIGS. 12(a), 12(b), 12(c) and 12(d), respectively.

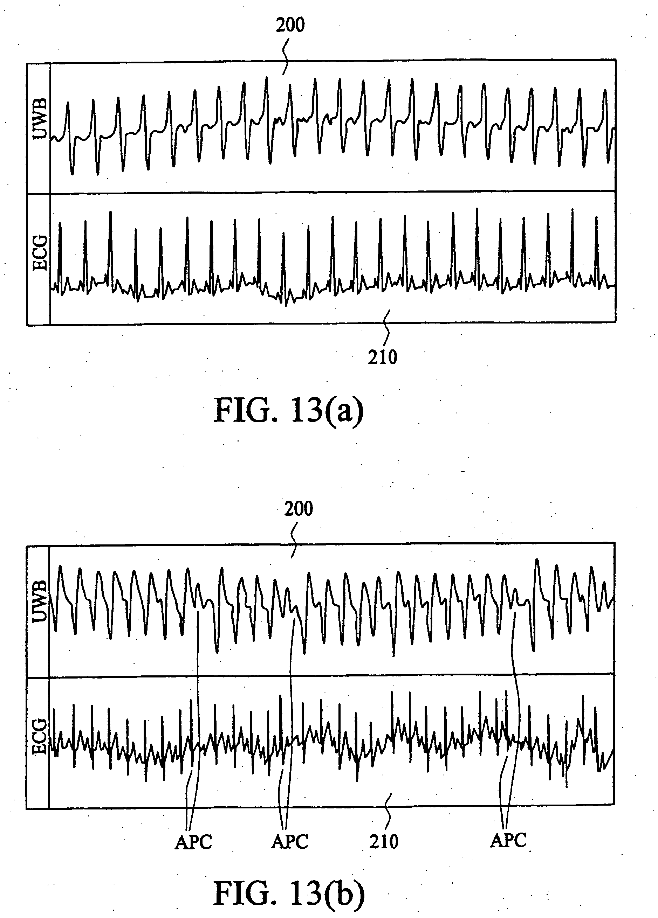

FIGs. 13(a) and FIG. 13(b) show the comparison of radial arterial signal waveform acquired by the monitoring apparatus 10 and the electrocardiogram signal waveform. As shown in FIG. 13(a) and FIG. 13(b), the performance of the present monitoring apparatus 10 was verified in a clinical setting. The subject's radial artery pulse signal 200, obtained using the monitoring apparatus 10, is compared to the subject's electrocardiogram 210. The result in FIG. 13(a) is obtained from a patient with normal heart rhythm, whereas that in FIG. 13(b) is obtained from a patient with symptom of arterial premature contraction (APC). Both results show excellent beat-to-beat match indicating that the monitoring apparatus can be used as a diagnostic tool for identification of patients with heart disease.

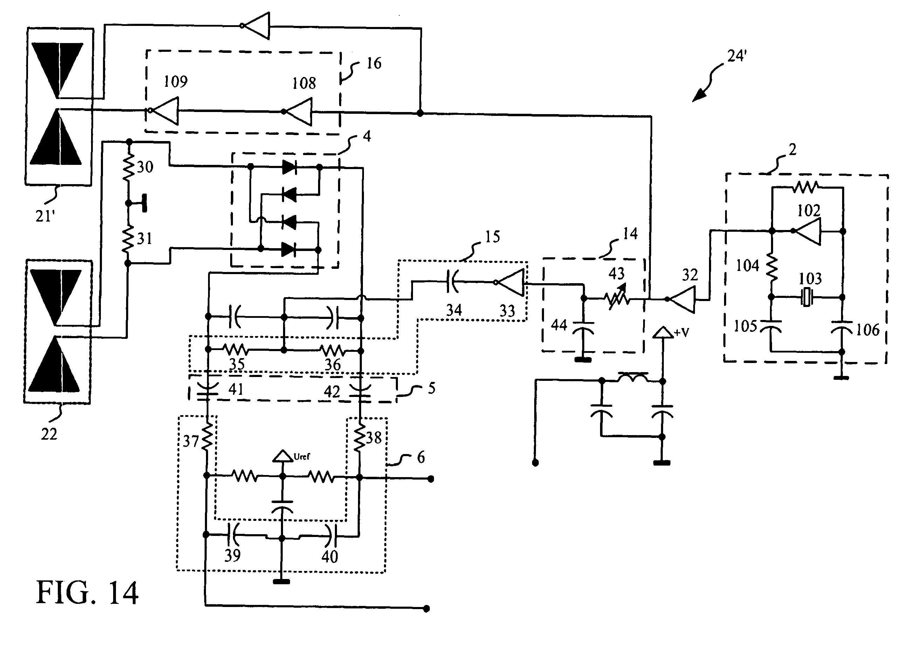

FIG. 14 shows a portion of the analog board 24' for the monitoring apparatus 10 according to another embodiment of the present invention. Compared to the circuit on the analog board 24 having a singled-ended output reception antenna 22 as shown in FIG. 5(a), the circuit on the analog board 24' in FIG. 14 has a differential output reception antenna 22', which can dramatically increase the bandwidth of the monitoring apparatus 10 and the amplitude of waveforms representing the physical movements of the body organ.

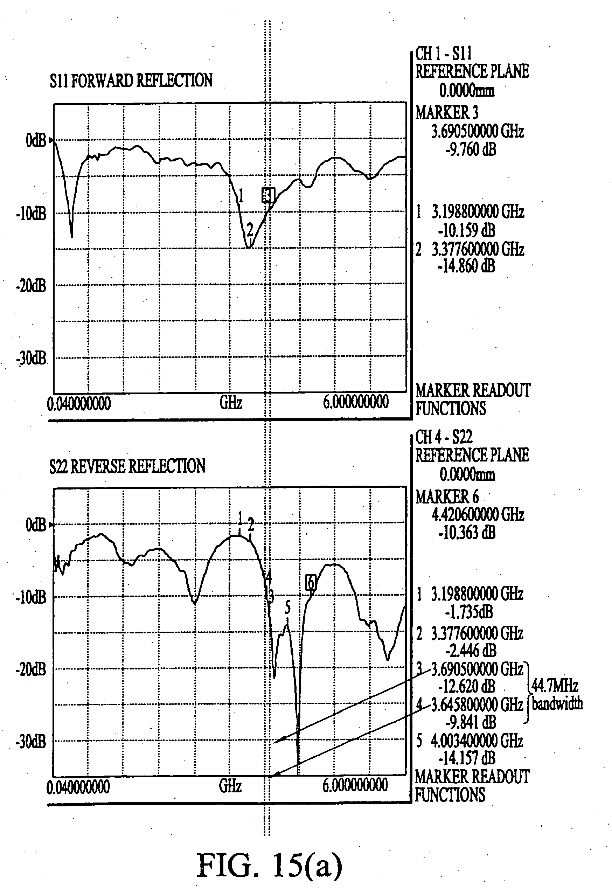

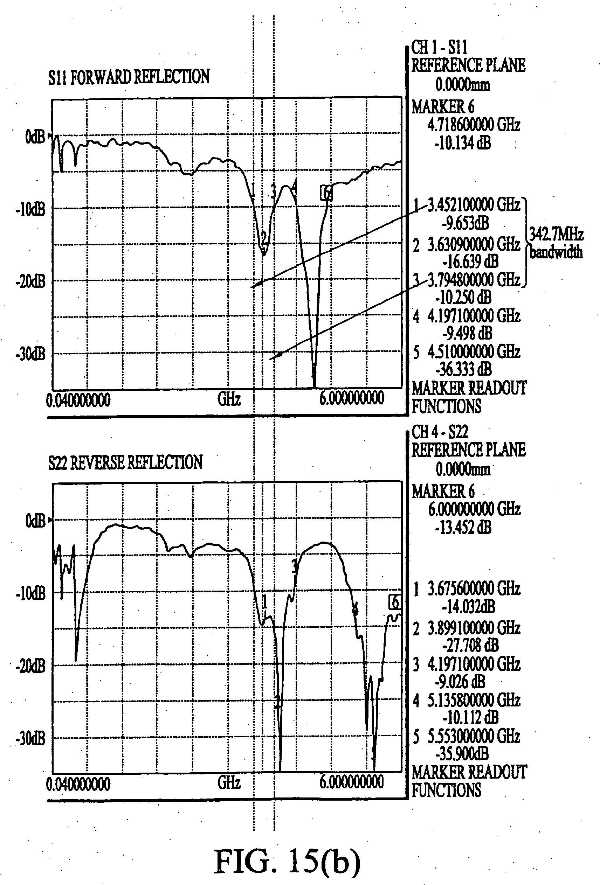

FIG. 15(a) shows the bandwidth of the monitoring apparatus 10 using the single-ended output reception antenna 22, and FIG. 15(b) shows the bandwidth of the monitoring apparatus 10 using the differential output reception antenna 22'. The bandwidth of the monitoring apparatus 10 using the single-ended output reception antenna 22 is only about 44.7 MHz as shown in FIG. 15(a), while the bandwidth of the monitoring apparatus 10 using the differential output reception antenna 22' is about 342.7 MHz as shown in FIG. 15(b). Obviously, the use of the differential output reception antenna 22' can increase the bandwidth of the monitoring apparatus 10 by about seven times.

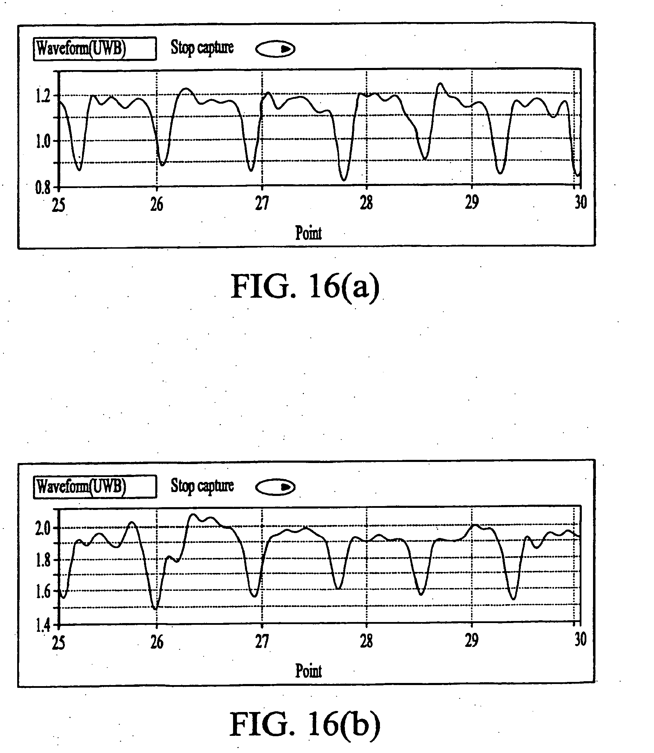

FIG. 16(a) shows the radial arterial signal waveform acquired by the present monitoring apparatus 10 using the single-ended output reception antenna 22, and FIG. 16(b) shows the radial arterial signal waveform acquired by the present monitoring apparatus 10 using the differential output reception antenna 22'. Obviously, the amplitude of the radial arterial signal waveform in FIG. 16(b) is about twice as large as that in FIG. 16(a). In other words, the use of the differential output reception antenna 22' can increase the amplitude of the physical movement signal of the body organ.

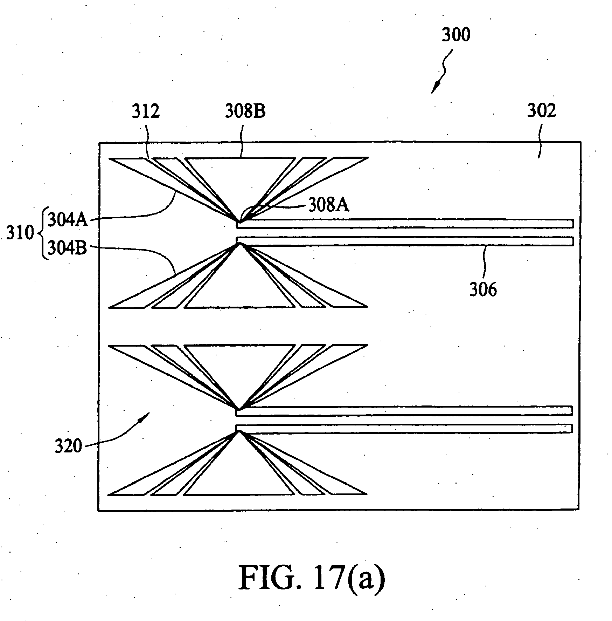

FIG. 17(a) illustrates a flexible slot antenna 300 according to another embodiment of the present invention. The flexible slot antenna 300 comprises a flexible substrate 302 preferably made of polyimide, a transmission antenna 310 positioned on the flexible substrate 302, and a reception antenna 320 substantially the same as the transmission antenna 310. The flexible substrate 302 facilitates to attach the flexible slot antenna 300 onto the human body. The transmission antenna 310 comprises a pair of metal blocks 304A, 304B, each of metal blocks 304A, 304B has at least one slot 312 such as a triangular slot positioned in a mirror manner and has a smaller lateral width at a feeding end 308A than at a free end 308B. In addition, the flexible slot antenna 300 further comprises a microstrip line 306 connected to the feeding end 308A of the metal block 304A. Preferably, the metal block 304A is triangular, fan-shaped or T-shaped, and the length of the microstrip line 306 is half of the wavelength of the ultra wide band electromagnetic wave.

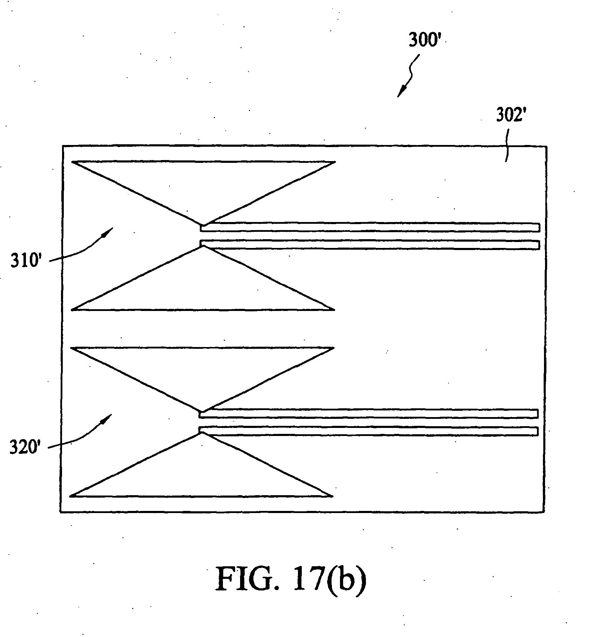

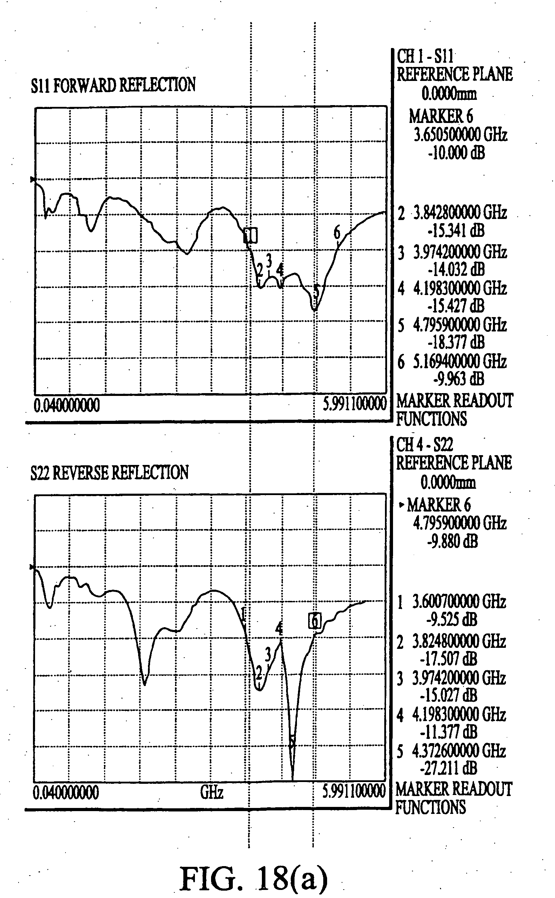

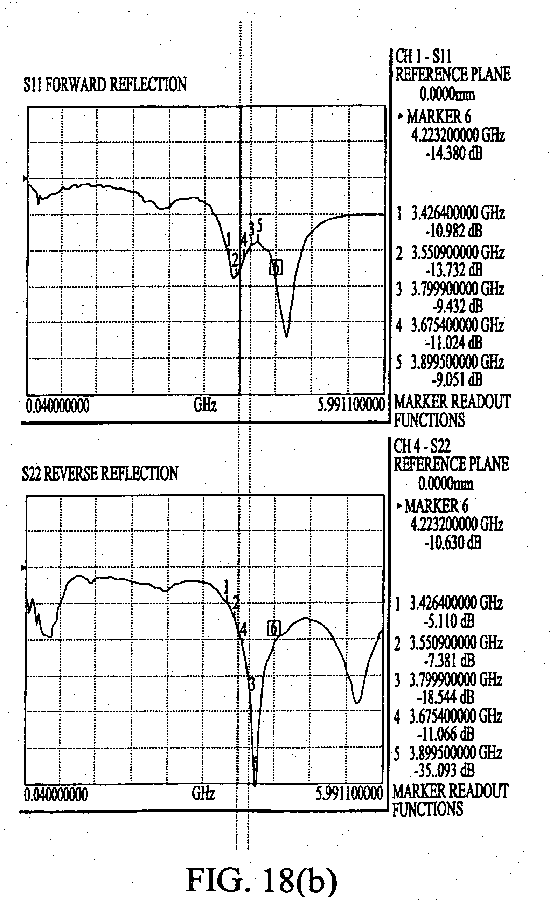

FIG. 18(a) shows the bandwidth of the monitoring apparatus 10 using the flexible slot antenna 300, and FIG. 18(b) shows the bandwidth of the monitoring apparatus 10 using the flexible antenna 300', which has a transmission antenna 310' and a reception antenna 320' without slots on a hard substrate 302', as shown in FIG. 17(b). The bandwidth of the monitoring apparatus 10 using the flexible slot antenna 300 is up to about 1.145 GHz as shown in FIG. 18(a), while the bandwidth of the monitoring apparatus 10 using the flexible antenna 300' is only about 120 MHz as shown in FIG. 18(b). Obviously, the use of the flexible substrate 302 and the slot 312 in the metal blocks 304A, 304B facilitate to increase the bandwidth of the monitoring apparatus 10 by about ten times.

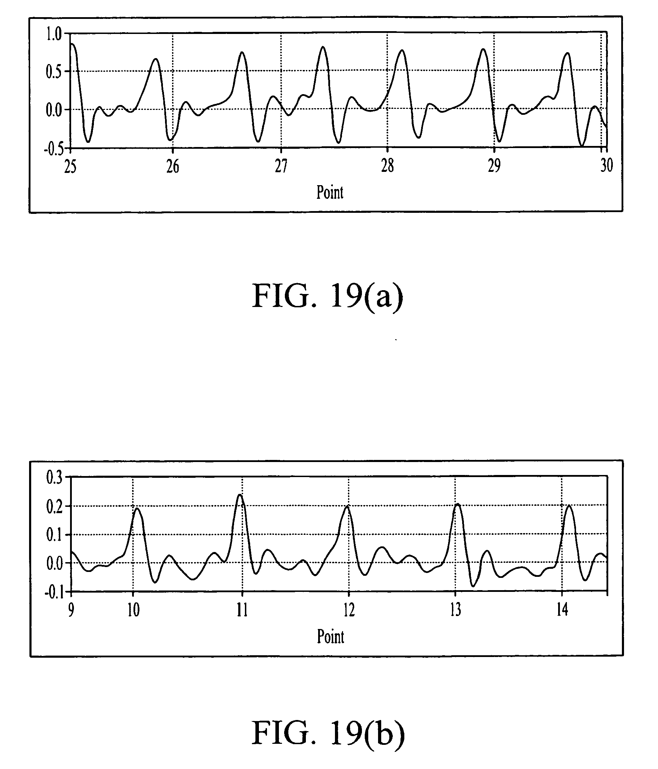

FIG. 19(a) shows the radial arterial signal waveform acquired by the present monitoring apparatus 10 using the flexible slot antenna 300, and FIG. 19(b) shows the radial arterial signal waveform acquired by the present monitoring apparatus 10 using the flexible antenna 300'. Obviously, the amplitude of the radial arterial signal waveform in FIG. 19 (a) is about four times as large as that in FIG. 19(b). In other words, the use of the flexible substrate 302 and the slot 312 in the metal blocks 304A, 304B facilitate to increase the amplitude of the physical movement signal of the body organ.

The above-described embodiments of the present invention are intended to be illustrative only. Numerous alternative embodiments may be devised by those skilled in the art without departing from the scope of the following claims.

**Claims**

1. A monitoring apparatus for physical movements of a body organ, comprising:

   a first pulse generator configured to produce ultra wideband electromagnetic probing pulses;
   a transmission antenna configured to radiate the probing pulses to a body organ;
   a reception antenna configured to receive the probing pulses scattered by the body organ;
   a second pulse generator configured to produce reference pulses;
   a balanced mixer having a first input port electrically connected to the second pulse generator and a second input port electrically connected to the reception antenna for producing a phase difference signal representing the physical movements of the body organ; and
   an analog-to-digital converter configured to convert the phase difference signal into a digital signal.

2. The monitoring apparatus for physical movements of a body organ according to Claim 1, further comprising:

   a clock generator;
   a delay line electrically connected to the clock generator, wherein the first pulse generator is based on the clock signal of the clock generator to produce the probing pulses, and the second pulse generator is electrically connected to the clock generator via the delay line and is based on the clock signal of the clock generator to produce the reference pulses.

3. The monitoring apparatus for physical movements of a body organ according to Claim 2, wherein the delay line consists of a variable resistor and a capacitor.

4. The monitoring apparatus for physical movements of a body organ according to Claim 1, wherein the transmission antenna and the reception antenna are selected from the group consisting of a loop antenna, a bow-tie antenna, a terminating half-wave antenna and a spiral antenna.

5. The monitoring apparatus for physical movements of a body organ according to Claim 1, wherein the reception antenna is a differential output antenna.

6. The monitoring apparatus for physical movements of a body organ according to Claim 1, wherein the transmission antenna comprises:

   a substrate;
   a pair of metal blocks having at least one slot positioned in each of the metal block in a mirror manner; and
   a microstrip line connected to a feeding end of the metal block.

7. The monitoring apparatus for physical movements of a body organ according to Claim 6, wherein the reception antenna is substantially the same as the transmission antenna.

8. The monitoring apparatus for physical movements of a body organ according to Claim 6, wherein each metal block has a smaller lateral width at the feeding end than at a free end.

9. The monitoring apparatus for physical movements of a body organ according to Claim 6, wherein each metal block is triangular, fan-shaped or T-shaped.

10. The monitoring apparatus for physical movements of a body organ according to Claim 6, wherein the slot is triangular.

11. The monitoring apparatus for physical movements of a body organ according to Claim 1, wherein the substrate is made of polyimide.

12. The monitoring apparatus for physical movements of a body organ according to Claim 1, further comprising:

at least one filter electrically connected to the balanced mixer; and
at least one amplifier electrically connected to the filter.

13. The monitoring apparatus for physical movements of a body organ according to Claim 1, further comprising:

a high pass filter electrically connected to the balanced mixer;
a first low pass filter electrically connected to the high pass filter;
a first amplifier electrically connected to the first low pass filter;
a second low pass filter electrically connected to the first amplifier; and
a second amplifier electrically connected to the second low pass filter.

14. The monitoring apparatus for physical movements of a body organ according to Claim 13, wherein the first amplifier is an instrumentation amplifier.

15. The monitoring apparatus for physical movements of a body organ according to Claim 13, wherein the second low pass filter is a fourth-order active low-pass Butterworth filter.

16. The monitoring apparatus for physical movements of a body organ according to Claim 1, wherein the analog-to-digital converter is embedded in a microcontroller.

17. The monitoring apparatus for physical movements of a body organ according to Claim 16, further comprising a transceiver electrically connected to the microcontroller for transferring serial data to a display device via a universal serial bus.

18. The monitoring apparatus for physical movements of a body organ according to Claim 16, further comprising a COM port driver electrically connected to the microcontroller for transferring data to the display device using an RS232 cable.

19. A monitoring apparatus for physical movements of a body organ, comprising:

a transmission antenna configured to ultra wideband electromagnetic radiate probing pulses to a body organ, wherein the transmission antenna comprises a pair of metal blocks having at least one slot positioned in each of the metal block in a mirror manner;
a reception antenna configured to receive the probing pulses scattered by the body organ; and
an analog signal processing unit having a first input port electrically connected to a reference pulse generator and a second input port electrically connected to the reception antenna for producing signals representing the physical movements of the body organ.

20. The monitoring apparatus for physical movements of a body organ according to Claim 19, wherein the transmission antenna and the reception antenna are positioned on a substrate made of polyimide.

21. The monitoring apparatus for physical movements of a body organ according to Claim 19, wherein each metal block has a smaller lateral width at the feeding end than at a free end.

22. The monitoring apparatus for physical movements of a body organ according to Claim 19, wherein each metal block is triangular, fan-shaped or T-shaped.

23. The monitoring apparatus for physical movements of a body organ according to Claim 19, wherein the slot is triangular.

24. The monitoring apparatus for physical movements of a body organ according to Claim 19, further comprising a microstrip line connected to a feeding end of the metal block.

25. The monitoring apparatus for physical movements of a body organ according to Claim 19, wherein the reception antenna is substantially the same as the transmission antenna

26. A method for acquiring physical movements of a body organ, comprising the following steps:

    radiating ultra wideband electromagnetic pulses to the body organ;
    measuring a phase difference signal between probing pulses scattered by the body organ and reference pulses; and
    acquiring the physical movements of the body organ based on the phase difference signal.

27. The method for acquiring physical movements of a body organ according to Claim 26, wherein the phase difference signal $\Delta\varphi$ is measured according to the following equation:

$$\Delta\varphi = \frac{4\pi \cdot \sqrt{\varepsilon} \cdot f}{c} \Delta d$$

$$\Delta d = V\,T_a$$

wherein $f$ represents the repetition frequency of the probing pulses, $c$ represents velocity of light and $\varepsilon$ represents the relative dielectric constant of the skin tissue. $V$ represents the velocity of the physical movement of the body organ toward where the probing pulses are radiated from, $T_a$ represents the time interval as the body organ moves a distance $\Delta d$

**Patentansprüche**

1. Beobachtungsvorrichtung für physische Bewegungen eines Körperorgans, die Folgendes umfasst:

    einen ersten Pulsgenerator, der konfiguriert ist, elektromagnetische Ultra-Breitband-Probepulse zu erzeugen;
    eine Übertragungsantenne, die konfiguriert ist, Probepulse zu einem Körperorgan zu senden;
    eine Empfangantenne, die konfiguriert ist, die von dem Körperorgan gestreuten Probepulse zu empfangen;
    einen zweiten Pulsgenerator, der konfiguriert ist, Referenzpulse zu erzeugen;
    einen ausgeglichenen Mischer ("balanced mixer"), der einen ersten Eingangsport umfasst, welcher elektrisch mit dem zweiten Pulsgenerator verbunden ist, und einen zweiten Eingangsport umfasst, der elektrisch mit der Empfangsantenne verbunden ist, um ein Phasendifferenzsignal zu erzeugen, welches die physischen Bewegungen des Körperorgans repräsentiert; und
    einen Analog/Digital-Wandler, der konfiguriert ist, das Phasendifferenzsignal in ein digitales Signal umzuwandeln.

2. Beobachtungsvorrichtung für physische Bewegungen eines Körperorgans nach Anspruch 1, die ferner Folgendes umfasst:

    einen Taktgenerator;
    eine Verzögerungsleitung, die elektrisch mit dem Taktgenerator verbunden ist, wobei der erste Pulsgenerator auf dem Taktsignal des Taktgenerators basiert, um die Probepulse zu erzeugen, und der zweite Pulsgenerator elektrisch über die Verzögerungsleitung mit dem Taktgenerator verbunden ist und auf dem Taktsignal des Taktgenerators basiert, um die Referenzpulse zu erzeugen.

3. Beobachtungsvorrichtung für physische Bewegungen eines Körperorgans nach Anspruch 2, bei der die Verzögerungsleitung aus einem variablen Widerstand und einem Kondensator besteht.

4. Beobachtungsvorrichtung für physische Bewegungen eines Körperorgans nach Anspruch 1, bei der die Übertragungsantenne und die Empfangsantenne aus der folgenden Gruppe von Antennen ausgewählt sind: Rahmenantenne, fliegenförmige Antenne ("bow-tie antenna"), eine terminierte Halbwellenantenne und eine Spiral-Antenne.

5. Beobachtungsvorrichtung für physische Bewegungen eines Körperorgans nach Anspruch 1, bei der die Empfangsantenne eine Differenzausgabe-Antenne ist.

6. Beobachtungsvorrichtung für physische Bewegungen eines Körperorgans nach Anspruch 1, bei der die Übertragungsantenne folgendes umfasst:

ein Substrat;
ein Paar von Metallblöcken, die mindestens einen Schlitz aufweisen, der spiegelbildlich in einem jeden der Blöcke angeordnet ist; und
eine Mikrostrip-Leitung, die mit einem Versorgungsende des Metallblocks verbunden ist.

7. Beobachtungsvorrichtung für physische Bewegungen eines Körperorgans nach Anspruch 6, bei der die Empfangsantenne im Wesentlichen gleich ist mit der Übertragungsantenne.

8. Beobachtungsvorrichtung für physische Bewegungen eines Körperorgans nach Anspruch 6, bei der ein jeder Metallblock an dem Versorgungsende eine geringere laterale Breite aufweist als an einem freien Ende.

9. Beobachtungsvorrichtung für physische Bewegungen eines Körperorgans nach Anspruch 6, bei der ein jeder Metallblock dreieckig, fächerförmig oder T-förmig ist.

10. Beobachtungsvorrichtung für physische Bewegungen eines Körperorgans nach Anspruch 6, bei der der Schlitz dreieckig ist.

11. Beobachtungsvorrichtung für physische Bewegungen eines Körperorgans nach Anspruch 1, bei der das Substrat aus Polyimid besteht.

12. Beobachtungsvorrichtung für physische Bewegungen eines Körperorgans nach Anspruch 1, die ferner Folgendes umfasst:

mindestens einen Filter, der elektrisch mit dem ausgeglichenen Mischer verbunden ist; und
mindestens einen Verstärker, der elektrisch mit dem Filter verbunden ist.

13. Beobachtungsvorrichtung für physische Bewegungen eines Körperorgans nach Anspruch 1, die ferner Folgendes umfasst:

einen Hochpassfilter, der elektrisch mit dem ausgeglichenen Mischer verbunden ist;
einen ersten Tiefpassfilter, der elektrisch mit dem Hochpassfilter verbunden ist;
einen ersten Verstärker, der elektrisch mit dem ersten Tiefpassfilter verbunden ist;
einen zweiten Tiefpassfilter, der elektrisch mit dem ersten Verstärker verbunden ist; und
einen zweiten Verstärker, der elektrisch mit dem zweiten Tiefpassfilter verbunden ist.

14. Beobachtungsvorrichtung für physische Bewegungen eines Körperorgans nach Anspruch 13, bei der der erste Verstärker einen Instrumentenverstärker ist.

15. Beobachtungsvorrichtung für physische Bewegungen eines Körperorgans nach Anspruch 13, bei der der zweite Tiefpassfilter ein aktiver Tiefpass-Butterworth-Filter der vierten Ordnung ist.

**16.** Beobachtungsvorrichtung für physische Bewegungen eines Körperorgans nach Anspruch 1, bei der der Analog/Digital-Wandler in einem Mikrocontroller eingebettet ist.

**17.** Beobachtungsvorrichtung für physische Bewegungen eines Körperorgans nach Anspruch 16, die ferner einen Transceiver umfasst, der elektrisch mit dem Mikrocontroller verbunden ist, um serielle Daten über einen Universal-Serial-Bus zu einer Anzeigevorrichtung zu übertragen.

**18.** Beobachtungsvorrichtung für physische Bewegungen eines Körperorgans nach Anspruch 16, die ferner einen COM-Port-Treiber umfasst, der elektrisch mit dem Mikrocontroller verbunden ist, um Daten unter Verwendung eines RS232-Kabels zu der Anzeigevorrichtung zu übertragen.

**19.** Beobachtungsvorrichtung für physische Bewegungen eines Körperorgans, die Folgendes umfasst:

eine Übertragungsantenne, die konfiguriert ist, elektromagnetische Ultra-Breitband-Probepulse zu einem Körperorgan zu senden, wobei die Übertragungsantenne ein Paar von Metallblöcken umfasst, die mindestens einen Schlitz aufweisen, die in dem jeweiligen Metallblock auf spiegelbildliche Weise angeordnet sind;
eine Empfangsantenne, die konfiguriert ist, die von dem Körperorgan gestreuten Probepulse zu empfangen; und
eine analoge Signalverarbeitungseinheit mit einem ersten Eingangsport, der elektrisch mit einem Referenzpulsgenerator verbunden ist, und einem zweiten Eingangsport, der elektrisch mit der Empfangsantenne verbunden ist, um Signale zu erzeugen, die die physischen Bewegungen des Körperorgans repräsentieren.

**20.** Beobachtungsvorrichtung für physische Bewegungen eines Körperorgans nach Anspruch 19, bei der die Übertragungsantenne und die Empfangsantenne auf einem Substrat angeordnet sind, welches aus Polyimid besteht.

**21.** Beobachtungsvorrichtung für physische Bewegungen eines Körperorgans nach Anspruch 19, bei der ein jeder Metallblock an dem Versorgungsende eine geringere laterale Breite aufweist als an einem freien Ende.

**22.** Beobachtungsvorrichtung für physische Bewegungen eines Körperorgans nach Anspruch 19, bei der ein jeder Metallblock dreieckig, fächerförmig oder T-förmig ist.

**23.** Beobachtungsvorrichtung für physische Bewegungen eines Körperorgans nach Anspruch 19, bei der der Schlitz dreieckig ist.

**24.** Beobachtungsvorrichtung für physische Bewegungen eines Körperorgans nach Anspruch 19, die ferner eine Mikrostrip-Leitung umfasst, die mit einem Versorgungsende des Metallblocks verbunden ist.

**25.** Beobachtungsvorrichtung für physische Bewegungen eines Körperorgans nach Anspruch 19, bei der die Empfangsantenne im Wesentlichen die gleiche ist wie die Übertragungsantenne.

**26.** Verfahren zum Erfassen physischer Bewegungen eines Körperorgans, das die folgenden Schritte umfasst:

Senden von elektromagnetischen Ultra-Breitband-Pulsen zu dem Körperorgan;
Messen eines Phasendifferenzsignals zwischen Probepulsen, die von dem Körperorgan gestreut wurden, und Referenzpulsen; und
Erfassen der physikalischen Bewegungen des Körperorgans basierend auf dem Phasendifferenzsignal.

**27.** Verfahren zum Erfassen physischer Bewegungen eines Körperorgans nach Anspruch 16, bei dem das Phasendifferenzsignal $\Delta\varphi$ gemäß der folgenden Gleichung gemessen wird:

$$\Delta\varphi = \frac{4\pi \cdot \sqrt{\varepsilon \cdot f}}{c} \Delta d$$

$$\Delta d = V T_a \, ,$$

wobei f die Wiederholungsfrequenz der Probepulse repräsentiert, c die Lichtgeschwindigkeit repräsentiert und ε die relative Dielektrizitätskonstante des Hautgewebes repräsentiert, V die Geschwindigkeit der physischen Bewegungen des Körperorgans in Richtung auf den Ort, von dem die Probepulse aus gesendet werden, repräsentiert und $T_a$ das Zeitintervall repräsentiert, in dem das Körperorgan sich um eine Strecke Δd bewegt.

**Revendications**

1. Dispositif de surveillance de mouvements physiques d'un organe du corps comprenant :

   un premier générateur d'impulsions agencé pour produire des impulsions électromagnétiques de sonde à très large bande ;
   une antenne d'émission agencée pour rayonner les impulsions de sonde vers un organe du corps;
   une antenne de réception agencée pour recevoir les impulsions de sonde dispersées par l'organe du corps ;
   un second générateur d'impulsions agencé pour produire des impulsions de référence ;
   un mélangeur équilibré ayant un premier accès d'entrée électriquement connecté au second générateur d'impulsions et un second accès d'entrée électriquement connecté à l'antenne de réception pour produire un signal de différence de phase représentant les déplacements physiques de l'organe du corps; et
   un convertisseur analogique-numérique agencé pour convertir le signal de différence de phase en un signal numérique.

2. Dispositif de surveillance de mouvements physiques d'un organe du corps selon la revendication 1, comprenant en outre :

   un générateur d'horloge ;
   une ligne à retard électriquement connectée au générateur d'horloge, le premier générateur d'impulsions étant basé sur le signal d'horloge du générateur d'horloge pour produire des impulsions de sonde et le second générateur d'impulsions étant électriquement connecté au générateur d'horloge par la ligne à retard et étant basé sur le signal d'horloge du générateur d'horloge pour produire des impulsions de référence.

3. Dispositif de surveillance de mouvements physiques d'un organe du corps selon la revendication 2, dans lequel la ligne en retard comprend une résistance variable et un condensateur.

4. Dispositif de surveillance de mouvements physiques d'un organe du corps selon la revendication 1, dans lequel l'antenne d'émission et l'antenne de réception sont choisies dans le groupe comprenant une antenne à boucle, une antenne en arc, une antenne à terminaison en demi onde et une antenne spirale.

5. Dispositif de surveillance de mouvements physiques d'un organe du corps selon la revendication 1, dans lequel l'antenne de réception est une antenne à sortie différentielle.

6. Dispositif de surveillance de mouvements physiques d'un organe du corps selon la revendication 1, dans lequel l'antenne d'émission comprend:

   un substrat ;
   deux blocs métalliques avec au moins une fente disposée dans chaque bloc métallique à la façon d'un miroir ; et
   une ligne microbande connectée à l'extrémité de l'alimentation du bloc métallique.

7. Dispositif de surveillance de mouvements physiques d'un organe du corps selon la revendication 6, dans lequel l'antenne de réception est sensiblement identique à l'antenne d'émission.

8. Dispositif de surveillance de mouvements physiques d'un organe du corps selon la revendication 6, dans lequel chaque bloc métallique a une plus petite largeur latérale au niveau de l'extrémité d'alimentation qu'au niveau d'une extrémité libre.

9. Dispositif de surveillance de mouvements physiques d'un organe du corps selon la revendication 6, dans lequel chaque bloc métallique est triangulaire, en forme d'éventail ou en forme de T.

10. Dispositif de surveillance de mouvements physiques d'un organe du corps selon la revendication 6, dans lequel la fente est rectangulaire.

11. Dispositif de surveillance de mouvements physiques d'un organe du corps selon la revendication 1, dans lequel le substrat est en polyimide.

12. Dispositif de surveillance de mouvements physiques d'un organe du corps selon la revendication 1, comprenant en outre :

   au moins un filtre électriquement connecté au mélangeur équilibré ; et
   au moins un amplificateur électriquement connecté au filtre.

13. Dispositif de surveillance de mouvements physiques d'un organe du corps selon la revendication 1, comprenant en outre :

   un filtre passe-haut électriquement connecté au mélangeur équilibré ;
   un premier filtre passe-bas électriquement connecté au filtre passe-haut ;
   un premier amplificateur électriquement connecté au premier filtre passe-bas ;
   un second filtre passe-bas électriquement connecté au premier amplificateur ; et
   un second amplificateur électriquement connecté au second filtre passe-bas.

14. Dispositif de surveillance de mouvements physiques d'un organe du corps selon la revendication 13, dans lequel le premier amplificateur est un amplificateur de mesure.

15. Dispositif de surveillance de mouvements physiques d'un organe du corps selon la revendication 13, dans lequel le second filtre passe-bas est un filtre actif passe-bas du quatrième ordre de Butterworth.

16. Dispositif de surveillance de mouvements physiques d'un organe du corps selon la revendication 1, dans lequel le convertisseur analogique-numérique fait partie d'un microcontrôleur.

17. Dispositif de surveillance de mouvements physiques d'un organe du corps selon la revendication 16, comprenant en outre un émetteur récepteur électriquement connecté au niveau du contrôleur pour transférer des données séries à un dispositif d'affichage par l'intermédiaire d'un bus série universel.

18. Dispositif de surveillance de mouvements physiques d'un organe du corps selon la revendication 16, comprenant en outre une commande d'accès COM électriquement connectée au microcontrôleur pour transférer des données vers le dispositif d'affichage en utilisant un câble RS232.

19. Dispositif de surveillance de mouvements physiques d'un organe du corps comprenant :

   une antenne d'émission agencée pour rayonner de façon électromagnétique des impulsions de sonde à très large bande vers un organe du corps, l'antenne d'émission comprenant deux blocs métalliques comportant au moins une fente disposée dans chaque bloc métallique à la façon d'un miroir ;
   une antenne de réception agencée pour recevoir les impulsions de sonde dispersées par 1'organe du corps ; et
   un module de traitement du signal analogique ayant un premier accès d'entrée électriquement connecté à un générateur d'impulsions de référence et un second accès d'entrée électriquement connecté à l'antenne de réception pour produire des signaux représentant les déplacements physiques de l'organe du corps.

20. Dispositif de surveillance de mouvements physiques d'un organe du corps selon la revendication 19, dans lequel l'antenne d'émission et l'antenne de réception sont disposées sur un substrat en polyimide.

21. Dispositif de surveillance de mouvements physiques d'un organe du corps selon la revendication 19, dans lequel chaque bloc métallique a une plus petite largeur latérale au niveau de l' extrémité d'alimentation qu'au niveau d'une extrémité libre.

22. Dispositif de surveillance de mouvements physiques d'un organe du corps selon la revendication 19, dans lequel chaque bloc métallique est triangulaire, en forme d'éventail ou en forme de T.

23. Dispositif de surveillance de mouvements physiques d'un organe du corps selon la revendication 19, dans lequel la fente est rectangulaire.

24. Dispositif de surveillance de mouvements physiques d'un organe du corps selon la revendication 19, comprenant en outre une ligne à microbande connectée à une extrémité de l'alimentation du bloc métallique.

25. Dispositif de surveillance de mouvements physiques d'un organe du corps selon la revendication 19, dans lequel l'antenne de réception est sensiblement identique à l'antenne d'émission.

26. Procédé d'acquisition de déplacements physiques d'un organe du corps comprenant les étapes suivantes :

   irradier un organe du corps par des impulsions électromagnétiques à très large bande ;
   mesurer un signal de différence de phase entre les impulsions de sonde dispersées par l'organe du corps et des impulsions de référence ; et
   acquérir les déplacements physiques de l'organe du corps à partir du signal de différence de phase.

27. Procédé d'acquisition de déplacements physiques d'un organe du corps selon la revendication 26, dans lequel le signal de différence de phase $\Delta\varphi$ est mesuré par l'équation suivante :

$$\Delta\varphi = \frac{4\pi \cdot \sqrt{\varepsilon} \cdot f}{c}\Delta d$$

$$\Delta d = V\ T_a$$

où f représente la fréquence de répétition des impulsions de sonde, c représente la vitesse de la lumière et $\varepsilon$ représente la constante diélectrique relative du tissu de la peau, V représente la vitesse du déplacement physique de l'organe du corps vers lequel les impulsions de sonde sont rayonnées, $T_a$ représente l'intervalle de temps pendant lequel l'organe du corps se déplace d'une distance $\Delta d$.

FIG. 1(a)

10

12

FIG. 1(b)

FIG. 1(c)

FIG. 2(a)

EP 1 803 396 B1

FIG. 2(b)

EP 1 803 396 B1

FIG. 3(a)

FIG. 3(b)

FIG. 4

EP 1 803 396 B1

FIG. 5(a)

FIG. 5(b)

EP 1 803 396 B1

EP 1 803 396 B1

FIG. 5(c)

FIG. 5(d)

FIG. 6(a)

FIG. 6(b)

FIG. 7(a)

FIG. 7(b)

EP 1 803 396 B1

FIG. 8(a)

FIG. 8(b)

EP 1 803 396 B1

FIG. 9

EP 1 803 396 B1

FIG. 10

FIG. 11

FIG. 12(b)

FIG. 12(d)

FIG. 12(a)

FIG. 12(c)

200

FIG. 13(a)

200

FIG. 13(b)

FIG. 14

FIG. 15(a)

FIG. 15(b)

FIG. 16(a)

FIG. 16(b)

FIG. 17(a)

FIG. 17(b)

S11 FORWARD REFLECTION

CH 1 - S11
REFERENCE PLANE
0.0000mm
MARKER 6
3.650500000 GHz
-10.000 dB

2  3.842800000 GHz
   -15.341 dB
3  3.974200000 GHz
   -14.032 dB
4  4.198300000 GHz
   -15.427 dB
5  4.795900000 GHz
   -18.377 dB
6  5.169400000 GHz
   -9.963 dB

0.040000000                    5.991100000

MARKER READOUT
FUNCTIONS

S22 REVERSE REFLECTION

CH 4 - S22
REFERENCE PLANE
0.0000mm
► MARKER 6
   4.795900000 GHz
   -9.880 dB

1  3.600700000 GHz
   -9.525 dB
2  3.824800000 GHz
   -17.507 dB
3  3.974200000 GHz
   -15.027 dB
4  4.198300000 GHz
   -11.377 dB
5  4.372600000 GHz
   -27.211 dB

0.040000000        GHz        5.991100000

MARKER READOUT
FUNCTIONS

FIG. 18(a)

S11 FORWARD REFLECTION

0.040000000        GHz        5.991100000

CH 1 - S11
REFERENCE PLANE
0.0000mm
▸ MARKER 6
4.223200000 GHz
-14.380 dB

1   3.426400000 GHz
-10.982 dB
2   3.550900000 GHz
-13.732 dB
3   3.799900000 GHz
-9.432 dB
4   3.675400000 GHz
-11.024 dB
5   3.899500000 GHz
-9.051 dB
MARKER READOUT
FUNCTIONS

S22 REVERSE REFLECTION

0.040000000        GHz        5.991100000

CH 4 - S22
REFERENCE PLANE
0.0000mm
▸ MARKER 6
4.223200000 GHz
-10.630 dB

1   3.426400000 GHz
-5.110 dB
2   3.550900000 GHz
-7.381 dB
3   3.799900000 GHz
-18.544 dB
4   3.675400000 GHz
-11.066 dB
5   3.899500000 GHz
-35..093 dB
MARKER READOUT
FUNCTIONS

FIG. 18(b)

44

FIG. 19(a)

FIG. 19(b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4489731 A
- US 5795300 A
- US 5573012 A